# EUROPEAN PATENT SPECIFICATION

(11) **EP 1 478 914 B1**
(45) Date of publication and mention of the grant of the patent: **01.06.2011**
(21) Application number: 02802349.7
(22) Date of filing: 25.10.2002
(51) Int. Cl.: G01N 1/20

(54) **A SAMPLING VALVE**
PROBENAHMEVENTIL
ROBINET D'ECHANTILLONNAGE

(30) Priority: 01.11.2001 IE 20010961
(43) Date of publication of application: 24.11.2004
(73) Proprietor: DRUHAN, Jim, Bunclody, County Wexford (IE)
(72) Inventor: DRUHAN, Jim, Bunclody, County Wexford (IE)
(74) Representative: O'Connell, Maura
(86) International application number: PCT/IE2002/000151
(87) International publication number: WO 2003/038406

(56) References cited:
- WO-A-01/77642
- US-A- 2 864 254
- US-A- 4 262 534
- US-A- 4 526 194
- US-A- 4 769 215

## Description

The present invention concerns a sampling valve, and in particular a sampling valve which permits, in the absence of an official or the like to oversee the process, the collection of a sample of grain, which sampling valve is capable of indicating whether or not said grain sample has been tampered with.

At present, the grain handled at granaries is subject to regular and rigorous sampling and grading for a large number of characteristics, depending on the particular grain being sampled, or alternatively on the use to be made of the grain. The grain may be sampled for moisture content, protein content, quantity of foreign material present, processing quality, end use quality, etc.

Accurate grain sampling is of importance to both the buyer and the producer of the grain. The information obtained from the sample is used to establish the quality and the value of the grain, thereby ensuring that producers get maximum returns for their grain according to the quality thereof. The grading of grain permits the implementation of bulk handling systems that help keep handling and transportation costs low, and the grain prices high, in a competitive global market. Through grading, grain with similar characteristics, but from different producers, may be combined into larger lots for more efficient handling and transportation.

As much of a countries grain travels long distances to its markets, the costs are increased, and it is therefore necessary to obtain the highest prices possible for the grain marketed. Grading also segregates grain into different quality levels to satisfy both foreign and domestic markets. In today's complicated markets, buyers may be on the other side of the globe from producers, and it is therefore necessary to have a standardised grading system to describe the quality of the grain to the customer. It is therefore clear that proper thought and attention must be given to the method of collection, the sample size, and the frequency of sample collection per unit volume of grain.

For these reasons, many countries require that a certified official be present during the sampling of grain, in order to ensure that the sample is not tampered with in any way.

It will be appreciated that this requirement creates numerous difficulties, in addition to adding to the expense of the sample process. The main difficulty arising from this arrangement is that the granary is dependent on the presence of the official to carry out sampling, thereby severely limiting the time frame within which the samples may be taken. This may then of course hamper subsequent or dependent operations.

US4526194 discloses a butterfly valve or a similar quarter-turn valve with a locking mechanism which permits the disc assembly to be rotated from the open position to the closed position, but prevents the disc assembly from being rotated back to the open position. The locking mechanism includes a cam carried on a rotatable stem supporting the valve disc assembly. The cam has a camming surface including a detent notch situated at a location corresponding to the open position of the disc assembly. A plunger, which is spring loaded into engagement with the camming surface, is urged into the detent notch when the disc assembly is rotated from the open to the closed position and prevents the disc assembly from being rotated back to the open position.

US4262534 discloses a sampling apparatus for fruit juice concentrates or other high viscosity liquids flowing in a pipeline. Small amounts of the liquid are collected automatically at preset intervals to provide a representative sample for quality control or other reasons. The sampling apparatus is designed to prevent leakage, maintain sanitation, provide reliable samples and prevent unauthorized tampering of the samples being taken.

It is therefore an object of the present invention to mitigate one or more of the above problems by providing a grain sampling valve which permits the tamper proof collection of a sample of grain.

According to a first aspect of the present invention there is provided a sampling valve for fluid tight transport of a material from an enclosure to a container, the valve comprising a body engagable with the container and engagable with the enclosure, the body including a passage which provides access between the enclosure and the container; an actuator in operative association with the body, the actuator being adapted for irreversible movement from a transport position in which the actuator occludes the passage to a first position in which the passage is exposed, and for irreversible movement from the first position to a second position in which the passage is again occluded; characterised in that the valve comprises a coupling engagable with the body when the actuator is in the transport position, locked to the body when the actuator is in the first position, and releasable from the body when the actuator is in the second position.

Preferably, the body includes an entry aperture and an exit aperture which therebetween define the passage, the exit aperture, in use, being in register with the container.

Preferably, the actuator is substantially cylindrical and is mounted substantially within the body and arranged for unidirectional rotation therein, the actuator including a through bore, which may, by rotation of the actuator, be located in register with both the entry aperture and the exit aperture simultaneously.

Preferably, the valve includes a release mechanism which, upon actuation, permits the actuator to be moved from the second position to the transport position, the release mechanism, in use, being operable only upon removal of the valve from the container.

Preferably, the valve includes means for certifying the location/time/date or the like at which the sample was taken.

Preferably, the certifying means comprises a chamber provided in the actuator, the chamber being adapted to carry a token; and a marking device mounted about the body and operable to apply a mark to the token, the chamber being accessible to the marking device only when the actuator is in the first position.

Preferably, the chamber is located, in use, internally of the body, the body being provided with an access aperture with which the chamber may be brought into register by rotation of the actuator into the first position.

Preferably, when the passage is occluded, an airtight seal is established between the valve and the container.

Preferably, the valve further comprises a lock adapted to selectively permit or prevent movement of the actuator.

According to a second aspect of the present invention there is provided a sampling apparatus comprising a valve according to any preceding claim, and a container engagable therewith.

Preferably, the body is releasably engagable with the container.

Preferably, the apparatus further comprises a seal which is securable, in use, between the valve and the container, the seal being adapted to prevent or indicate removal of the valve from the container.

Preferably, the apparatus comprises a coupling in fluid communication with the enclosure, the coupling being engagable with the body when the actuator is in the transport position, locked to the body when the actuator is in the first position, and releasable from the body when the actuator is in the second position.

Preferably, the coupling and the actuator are interengagable such as to permit the coupling to be locked to the body when the actuator is in the first position.

Preferably, the actuator includes a key which projects outwardly of the body, and the coupling includes a corresponding keyway in which the key is located when the coupling is engaged to the body.

Preferably, the coupling is substantially cylindrical such as, in use, to be engaged substantially about the body in order to prevent access to the body.

Preferably, the coupling has an open end through which, in use, the body is inserted, and a closed end in which the keyway is located.

Preferably, the coupling covers the access aperture when the coupling is engaged to the body, the marking device comprising a stamp mounted to the coupling about an opening therein, which opening is in register with the access aperture when the coupling is engaged to the body, such that the stamp may be passed through the access aperture and contact the token, in order to apply the mark to the token.

Preferably, the container is in the form of a hollow truncated cone.

Preferably, the container is substantially transparent or opaque.

As used herein, the term "sampling" is intended to mean the collection of a material, in particular a random collection from a larger volume of the material, preferably for the purposes of testing/grading that sample to determine certain characteristics of the larger volume of material.

As used herein, the term "material" is intended to mean any substance, composition, etc, whether solid or fluid, which is sufficiently flowable to be capable of being poured, in particular into a container for the collection of the material.

As used herein, the term "irreversible" is intended to mean the confinement of movement to one direction, without being capable of movement in the reverse direction.

As used herein, the term "grain" is intended to mean, but is not limited to, wheat, barley, corn, rye, oats, flaxseed, sorghum, soybeans, mixed grain, and any other food grains, feed grains, and oilseeds for which standards have been established. As used herein, the term "seal" is intended to mean either a tamper proof seal preventing separation of two or more components, or a tamper evident seal which is adapted to indicate when two or more components have been separated.

The present invention will now be described with reference to the accompanying drawings, in which;

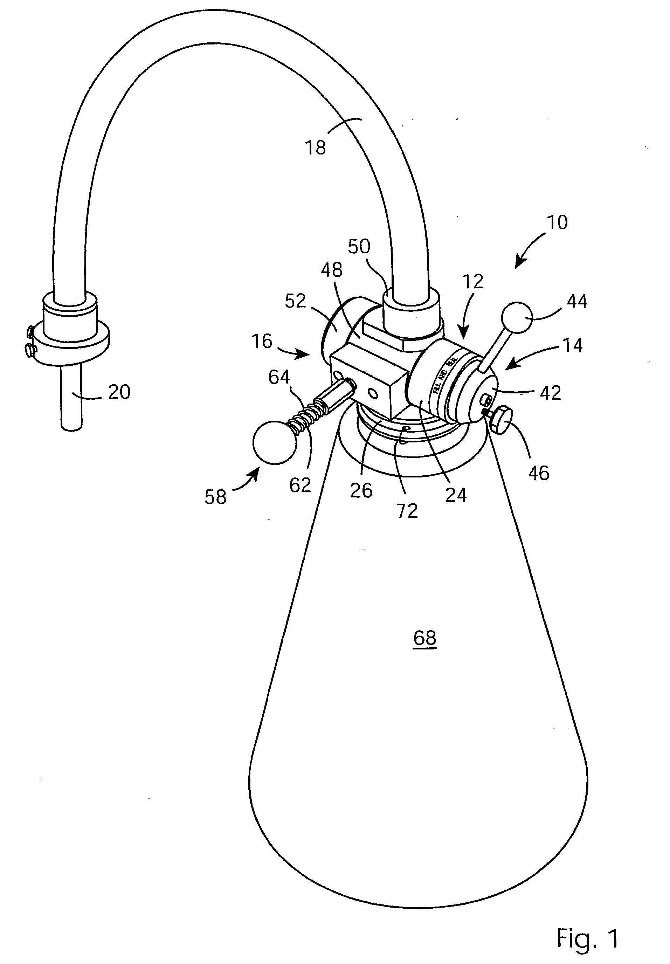

Figure 1 illustrates a perspective view of a sampling valve according to the present invention, the valve being mounted to a container to form an apparatus for collecting a sample of, in particular, grain, the valve further having a feed tube connected thereto;

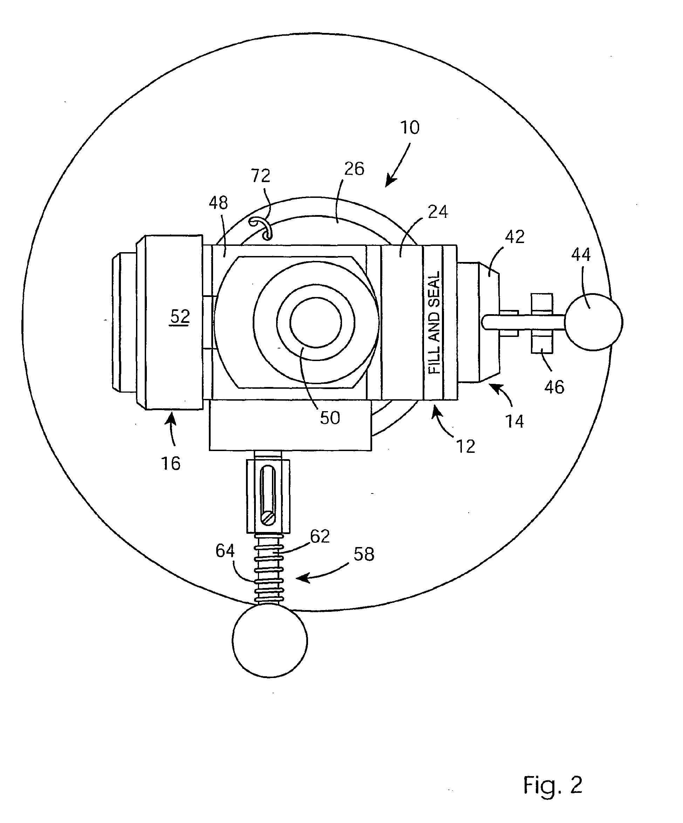

Figure 2 illustrates a plan view of the valve and apparatus of Figure 1, in which the feed tube has been removed for the purposes of clarity;
Figure 3 illustrates a side elevation of a body, in addition to an actuator located substantially within the body, both of which form part of the valve of the present invention;
Figure 4 illustrates a perspective view of the underside of a coupling for connection, in use, to the valve of the present invention;
Figure 5 illustrates a perspective view of the body and the actuator of Figure 3; and
Figure 6 illustrates a perspective view of the valve of the present invention, again secured about the container.

Referring now to the accompanying drawings, there is illustrated a sampling valve, generally indicated as 10, for use in permitting the tamper-evident sampling -of grain (not shown), or indeed any other flowable material, as will be appreciated from the following description of the invention. The valve 10 consists primarily of a body 12, and an actuator 14 disposed substantially within the body 12. The body 12, in use, is engaged to a container 68 to form a sampling apparatus, into which container 68 a sample of grain may be deposited for subsequent testing. The body 12 and the actuator 14, in combination, act as a gate which may be selectively actuated to permit or prevent the passage of the grain into the container 68, as will be described in detail hereinafter. A coupling 16 is therefore provided to secure a feed tube 18 to the valve 10, through which feed tube 18 the sample of grain is channelled. The feed tube 18 is preferably arranged, in use, such that grain migrates downwardly therethrough under the influence of gravity. It will however be appreciated that the grain could be driven through the feed tube 18 under pneumatic pressure or the like, or by any other suitable means. In use, the feed tube 18 is connected to a store (not shown) of grain from which the sample is to be taken. The feed tube 18 is preferably provided with a spoon 20 at the free end thereof, the spoon 20 being conventionally arranged and dimensioned to funnel the grain into the feed tube 18. However, any other suitable equivalent for the spoon 20 could be used, which was capable of directing grain into the feed tube 18.

The spoon 20 is therefore permanently secured and sealed within the store of grain, such that the spoon 20 cannot be externally accessed, thereby preventing the possibility of tampering with the sample as it enters the feed tube 18. Similarly, the feed tube 18 is permanently secured to the coupling 16, in order to prevent tampering with the sample prior to passing through the coupling 16. The spoon 20 is capable of being rotated about a longitudinal axis therefore, in order to permit or prevent the funnelling of grain into the feed tube 18, as is well known. It will therefore be understood that the coupling 16, in use, is permanently connected to the store of grain, via the feed tube 18, whereas the body 12 may be uncoupled from the coupling 16, in order to allow the container 68, plus the sample of grain, to be transferred elsewhere for testing.

Thus, in any given granary or store house (not shown) each individual store or vat of grain would have a feed tube 18 projecting therefrom, preferably at some form of dedicated sampling station (not shown), the feed tube 18 having the coupling 16 disposed at the free end thereof. Thus, when it is required to take a sample from a particular vat or store of grain, a worker would simply attend the sampling station with an empty, pre-prepared container 68 having the valve 10 secured thereto, as will be described in detail hereinafter, connect same to the feed tube 18 via the coupling 16, and collect a sample therein. The sample within the container 68 can then be sent for independent analysis/testing as required.

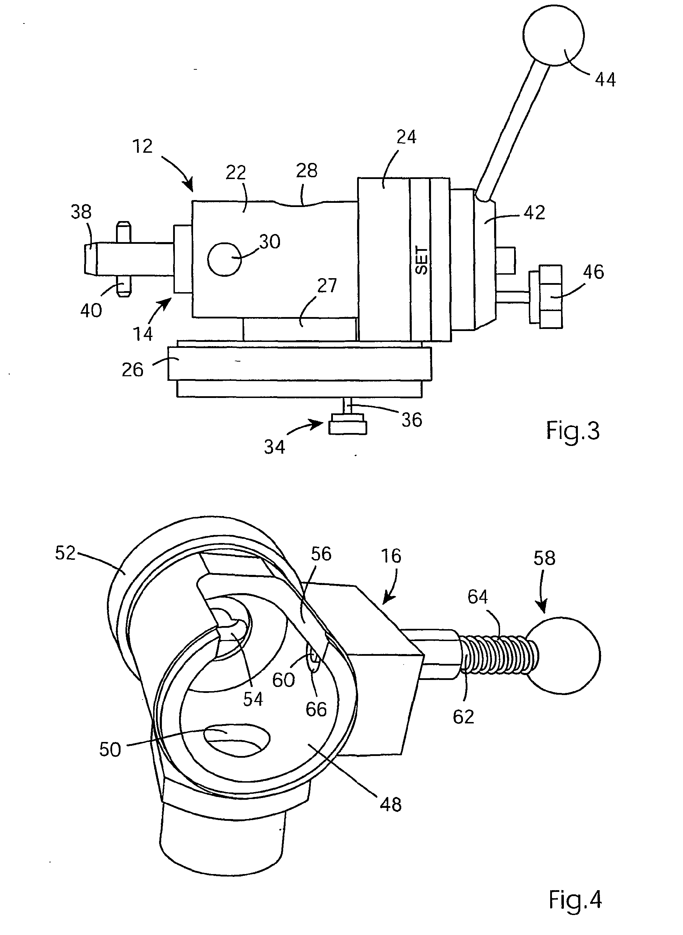

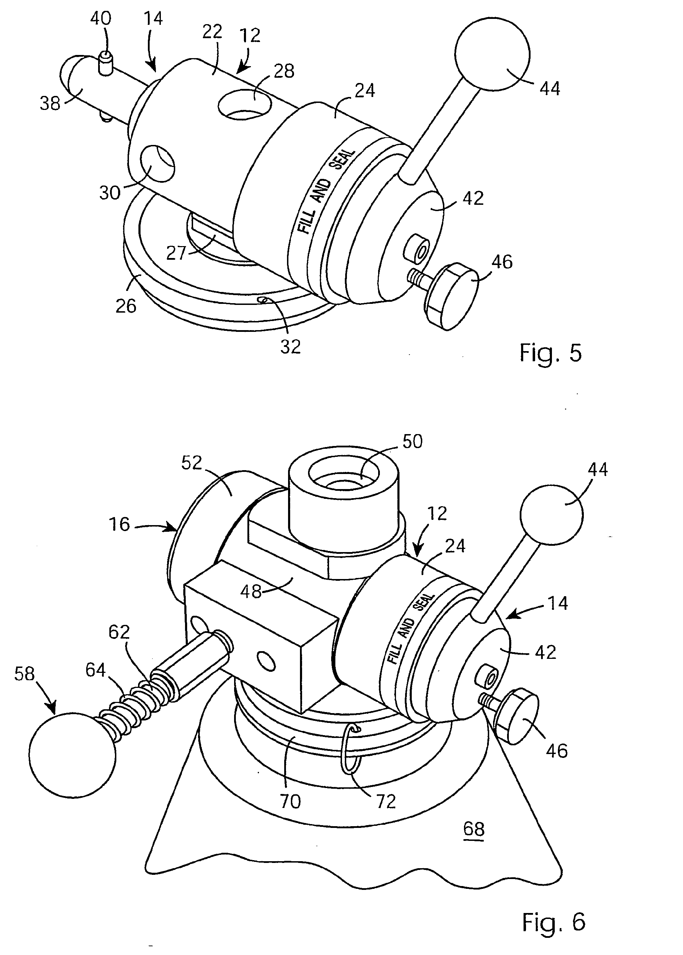

Turning then to the specific configuration and operation of the body 12, particular reference is made to Figure 3 and Figure 5 of the drawings. The body 12 consists of a barrel 22 which is hollow and cylindrical, and includes an enlarged shoulder 24, which is again hollow and cylindrical. The barrel 22 is mounted to a base 26 by means of a neck 27 and the inner edge (not shown) of the base 26 is threaded to permit the body 12 to be mounted to the container 68, such as to form a closure thereon. The closure could of course be effected by any other suitable means, preferably one that provides an air tight seal between the base 26 and the container 68. Thus, in order for the grain to be deposited within the container 68, the grain must either pass through the body 12, or alternatively the body 12 must be removed from the container 68. In order to prevent, in use, the removal of the body 12 from the container 68 to facilitate tampering with the sample, the base 26 is provided with a fixing bore 32 which, when the base 26 is secured to the container 68, is in register with a similar bore (not shown) in a rim 70 of the container 68. A looped seal 72 is therefore passed through the base 26 and the rim 70, thereby preventing removal of the body 12 from the container 68 without breaking the seal 72. The seal 72 therefore acts as a tamper-evident indicator. Thus it will be apparent that the looped seal 72 could be replaced with any other suitable form of tamper evident seal which would serve to indicate the breaking thereof during removal of the body 12 from the container 68.

In order to permit the passage of grain through the body 12 and into the container 68, an inlet 28 provides access to the interior of the barrel 22. In addition, the barrel 22 is provided with an outlet (not shown) oppositely disposed to the inlet 28, which outlet is in register with the neck 27. The neck 27 is itself hollow, and in addition opens onto the underside of the base 26, such that a passage is established to the interior of the container 68, by means of the inlet 28, the interior of the barrel 22, the outlet (not shown), and the hollow neck 27.

The actuator 14 is provided and adapted to selectively permit or prevent the passage of grain through the body 12. The actuator 14 is of solid cylindrical form and, in use, is permanently secured within the barrel 22 of the body 12, such as to occupy the interior thereof The actuator 14 includes an end cap 42 which abuts against the shoulder 24, and a nose 38 which projects outwardly of the barrel 22. The nose 38 includes a key in the form of a pin 40, the function of which will be described in detail hereinafter. The actuator 14 is adapted for unidirectional or irreversible rotation within the barrel 22 which, in the preferred embodiment illustrated, is clockwise when viewing the end cap 42 face on. The limitation to unidirectional motion may be achieved by a large number of conventional methods, and in the preferred embodiment illustrated, is achieved by the use of a conventional ratchet bearing (not shown) mounted within the shoulder 24. The reason for this limitation to unidirectional motion will become apparent from the following description of the operation of the valve 10.

It will be appreciated that as the actuator 14 is seated within the barrel 22, the actuator 14 acts as an impediment to the passage of grain through the body 12 and into the cylinder 68. However, the actuator 14 is provided with a through bore (not shown) which may, by rotation of the actuator 14, be brought into register with the inlet 28 and the outlet (not shown) simultaneously, thereby permitting passage of the grain through the body 12. It will therefore be appreciated that it is the function of the actuator 14 which is important to the operation of the valve 10, as opposed to the exact physical configuration of the preferred embodiment illustrated. Thus the shape and configuration of the actuator 14 could be altered, provided that the function remained of being adapted to selectively permit or prevent the passage of grain through the body 12.

However, it will be appreciated that in order to prevent tampering with the sample of grain, it must be possible to verify that the container 68 has been filled with grain which has passed through the feed tube 18 and the coupling 16. The valve 10 is therefore provided with a number of features and functions which operate in tandem in order to permit the verifiable collection of a sample of grain.

It will be appreciated that twice in a single 360° revolution of the actuator 14 within the barrel 12, will the through bore (not shown) be aligned with the inlet 28 such as to permit passage of the grain through the body 12 to the container 68. The actuator 14 is provided with a handle 44 projecting from the end cap 42, which handle 44 is provided to facilitate rotation of the actuator 14. Thus the handle 44 could be of any suitable form, or indeed the end cap 42 could be provided with some form of handgrip (not shown) formed integrally therewith, permitting the omission of the handle 44.

For the following operational description, the handle 44 will be used as a positional reference for the purposes of describing the operation of the actuator 14. Therefore, when the handle 44 is pointing vertically upwards, the actuator 14 will hereinafter be described as being disposed at 0°. When the handle 44 is turned one quarter of a rotation clockwise, the actuator 14 will be said to be disposed at 90°, when turned through half a revolution, 180° and when turned through three-quarters of a revolution, at 270°. The through bore (not shown) located within the actuator 14 is oriented such as to be in register with the inlet 28 when the actuator 14 is at both 0° and 180° as herein before described.

However, the valve 10 is adapted such that, upon reaching the 90° position, the actuator 14 becomes locked in place, with further rotation only possible by operation of a release mechanism 34 located under the base 26, as can be clearly seen in Figure 3. Again, locking of the actuator 14 in the 90° position may be achieved by any number of conventional means, but in the preferred embodiment illustrated, is as follows. The release mechanism 34 includes a rod 36 which passes upwardly through the base 26, the neck 27, and the barrel 22 such as to be in register with the actuator 14. The release mechanism 34 is spring loaded towards the actuator 14 such that the rod 36 is biased into engagement with the actuator 14. The actuator 14 is provided with a correspondingly shaped recess (not shown) which, upon rotation-of the actuator 14 to the 90° position, is in register with the rod 36 such that the rod 36 is forced into the recess (not shown), thereby preventing further rotation of the actuator 14 without withdrawal of the rod 36 from the recess (not shown). Thus, when locked at 90°, the actuator 14 occludes the passage through the body 12, thereby preventing tampering with any sample located within the container 68. In order to permit further rotation of the actuator 14 such as to again locate the through bore (not shown) in register with the inlet 28, the body 12 must be removed from the container 68 to gain access to the release mechanism 34. This would therefore require the seal 72 to be broken, thereby invalidating the sample within the container 68.

Furthermore, in use, the container 68 is provided to the granary or the like with the body 12 mounted thereon, the seal 72 in place, and the actuator 14 pre-set at the 270° position such that the passage through the body 12 is occluded. Therefore, due to the limitation to unidirectional rotation of the actuator 14, and the above described locking of the actuator 14 at the 90° position, it is possible for the sample to be taken only when the actuator 14 is at 0°. Thus, the actuator 14 is adapted to permit the body 12 to be inserted into engagement with the coupling 16 when the actuator 14-is at 270°, and to be locked within the coupling 16 when the actuator 14 is at 0°. This is achieved by the interaction of the nose 38 and the pin 40 of the actuator 14 with a corresponding keyway 54 formed in the coupling 16 as hereinafter described.

The coupling 16 comprises a sleeve 48 shaped and dimensioned for receiving the body 12, the sleeve 48 being provided with a mouth 56 which, in use, is seated about the neck 27. The coupling 16 includes a port 50 which passes through the sleeve 48 and to which the feed tube 18 is permanently secured. When the body 12 is seated within the coupling 16, the port 50 is in register with the inlet 28, in order to permit the flow of grain from the feed tube 18, through the body 12, and into the container 68. The coupling 16 is further provided with an end plate 52 in which is located the keyway 54. The keyway 54 is oblong in shape, and has a length just greater than that of the pin 40. A recess (not shown) is provided behind the keyway 54 such that the nose 38 and pin 40 may be inserted through and past the keyway 54. It will of course be understood that if the actuator 14 is then rotated such that the pin 40 is no longer aligned with the keyway 54, the nose 38 and the pin 40 may not be withdrawn from the keyway 54, and thus the body 12 cannot be withdrawn from the coupling 16.

The keyway 54 is orientated relative to the coupling 16 such that the body 12 may be inserted within the coupling 16 when the actuator 14 is disposed at 270°, and thus when passage through the body 12 is occluded. If the actuator 14 is then rotated to 0° such as to permit the passage of grain through the body 12 into the container 68, the pin 40 will be disposed, beyond the keyway 54, such as to prevent removal of the body 12 from the coupling 16. In order to permit the body 12 to be removed from the coupling 16, once the sample has been taken, the actuator 14 must, due to the limitation to unidirectional rotation, be rotated through to 90°, such that the pin 40 is again aligned with the keyway 54. As herein before described, upon reaching the 90° position, the passage through the body 12 will again be occluded, and the actuator 14 becomes locked in position. The body 12 may therefore be removed from the coupling 16, but as passage through the body 12 is now occluded, and the actuator 14 locked in position, the sample of grain within the container 68 cannot now be tampered with without removal of the body 12 from the container 68. This, as previously described, would break the seal 72, thereby invalidating the sample within the container 68.

From the above description of the interaction of the pin 40 and keyway 54 combination, it will be apparent that these two components could be replaced by any other functional equivalent, and that the pin 40 and keyway 54 combination is merely the preferred means of achieving the desired effect, while maintaining simplicity of design and operation.

It will of course be apparent that, in order to falsify a sample, the actuator 14 could simply be rotated to the 0° position, and a quantity of grain poured through the body 12 into the container 68, without the body 12 having to be seated within the coupling 16. The valve 10 must therefore be capable of verifying that the sample of grain within the container 68 was actually deposited while the body 12 was located and locked within the coupling 16. A marking device 58 is therefore mounted to the coupling 16 such as to be mounted, in use, about the body 12. The marking device 58 consists of a spring 64 loaded bar 62 which is mounted to the sleeve 48 in register with an opening 66 therein, as can be clearly seen in Figure 4. Located at the free end of the bar 62 is a stamp 60 which is positioned, in use, adjacent the opening 66. It will thus be apparent that the bar 62 may be pushed inwardly towards the coupling 16, against the action of the spring 64 such as to cause the stamp 60 to project inwardly of the sleeve 48. The body 12 is provided with an access aperture 30 in the barrel 22, which access aperture 30 is located in register with the opening 66 when the body 12 is seated within the coupling 16. The actuator 14 is provided with a chamber (not shown) which may, by rotation of the actuator 14, be brought into register with the access aperture 30. The chamber (not shown) is positioned on the actuator 14 such as to be in register with the access aperture 30 when the actuator 14 is at 0°. The chamber (not shown) in the actuator 14 is adapted to carry a token or similar indicia (not shown) which may be in the form of a cardboard disc or the like, to which token (not shown) the stamp 60 may apply a certifying mark. Thus, when the body 12 is located within the coupling 16, and the actuator 14 is disposed at 0° such as to lock the body 12 within the coupling 16, the token (not shown) will be in register with the access aperture 30. The marking device 58 may therefore be depressed such as to force the stamp 60 through the access aperture 30 to engage the token (not shown), thereby applying the certifying mark thereto.

The stamp 60 preferably embosses a mark or symbol into the token (not shown), thereby obviating the requirement for ink or the like to be continually applied to the stamp 60. It is of course possible that the entire marking device 58 could be replaced with, for example, an ink or laser jet printing head (not shown) or the like. However, such an arrangement would add cost and complexity to the valve 10, and would in addition reduce the reliability and/or robustness of the valve 10. Due to the preferred intended use of the valve 10, for example in granaries or the like, where significant levels of dust and similar air borne particles will be present, it is of course highly desirable that the configuration of the valve 10 remain as simple as possible, and preferably solely mechanically operable.

Once the sample has been taken, the actuator 14 is rotated to the 90° position in order to allow the body 12 to be removed from the coupling 16, and this rotation will therefore draw the chamber (not shown) and the token (not shown) located therein out of register with the access aperture 30 such that the token (not shown) cannot subsequently be tampered with. The only way that the token (not shown) can then be recovered for verification is to rotate the actuator 14 back to the 0° position, which would require the actuator 14 to be unlocked by operation of the release mechanism 34, thereby requiring the seal 72 to be broken and the sample invalidated.

Therefore, in use, the container 68 must be independently prepared prior to being sent to the granary at which the sample is to be taken. Thus, before applying the body 12 and the seal 72 to the container 68, the token (not shown) must be deposited in the chamber (not shown) within the actuator 14. The actuator 14 is therefore initially positioned at 0°, and the token (not shown) passed through the access aperture 30 into the chamber (not shown) which is located in register with the access aperture 30. The actuator 14 is then rotated to the 90° position, wherein the actuator 14 becomes locked in position by means of the release mechanism 34. The release mechanism 34 is then operated to permit the actuator 14 to be rotated through to the 270° position, at which position the actuator 14 must be maintained in readiness for insertion into the coupling 16. In this position, the token (not shown) located within the chamber (not shown) is out of register with the access aperture 30 and can not therefore be tampered with or falsely stamped prior to a sample being taken. The base 26 of the body 12 is then threaded onto the container 68, and the seal 72 affixed between the body 12 and the container 68. At this stage the container 68 is ready to be sent to the granary for taking a sample of grain or the like, as herein before described.

In order to prevent the inadvertent rotation of the actuator 14 from the 270° position during the handling or transport of the container 68 to or within the granary, the valve 10 is provided with a lock 46 which is threaded into engagement with the end cap 42 of the actuator 14. With the actuator 14 in any position, the lock 46 may be threaded inwardly.such as to engage the ratchet bearing (not shown) disposed between the actuator 14 and the shoulder 24, in order to prevent rotation of the actuator 14. Thus, when the actuator 14 is disposed at the 270° position, the lock 46 may be actuated until the container 68 has reached its destination, wherein the lock 46 may be disengaged to again permit rotation of the actuator 14. It will be immediately apparent that the lock 46 could be altered in shape/configuration/operation, again provided that the function thereof is embodied by any suitable equivalent used, namely that rotation of the actuator 14 can be selectively locked and released.

It is preferable that all of the components of the valve 10, or at least those components which may come into contact with the grain during the sampling thereof, are manufactured from stainless steel or the like, in order to prevent contamination of the sample collected. Furthermore, it is preferable that when the actuator 14 is disposed at 270°, subsequent to the sample being taken, an airtight seal is provided with the container 68 in order to prevent the ingress of moisture to the grain collected within the container 68, such as to maintain the integrity of the sample taken.

It will also be appreciated that the container 68 is not intended to be limited to the particular shape and dimensions shown for the preferred embodiment illustrated. The substantially conic shape has been chosen simply so that the container 68 may be stood upright without any support, for example while a sample is being collected therein, and during transport and/or storage of either the filled or empty container 68. In addition, as the walls of the container 68 flare outwardly from the base 26, the container 68 does not present any impediment or restriction to the flow of grain into the container 68 during the collection of the sample.

The container 68 is also preferably formed from any suitable material, preferably plastic, thereby providing resilience and wear resistance, both desirable properties due to the use of the container 68 in an industrial environment. The container 68 may also be substantially transparent or opaque, in order to permit the visual confirmation of the presence or otherwise of a sample therein.

It will be appreciated that, were the valve 10 permanently secured to the container 68, both the seal 72 and the release mechanism 34 could be omitted, thereby permitting a single use only of the resulting apparatus (not shown). The container (not shown) would therefore have to be broken open to permit access to the sample. Although such an arrangement would doubtless be a more costly approach, the use of plastic or the like for the apparatus (not shown) would reduce the cost, and permit recycling of the apparatus following retrieval of the sample.

## Claims

1. A sampling valve (10) for fluid tight transport of a material from an enclosure to a container, the valve comprising a body (12) engagable with the container and engagable with the enclosure, the body including a passage which provides access between the enclosure and the container; an actuator (14) in operative association with the body, the actuator being adapted for irreversible movement from a transport position in which the actuator occludes the passage to a first position in which the passage is exposed, and for irreversible movement from the first position to a second position in which the passage is again occluded; **characterised in that** the valve comprises
a coupling (16) engagable with the body when the actuator is in the transport position, locked to the body when the actuator is in the first position, and releasable from the body when the actuator is in the second position.

2. A valve according to claim 1 wherein the body includes an entry aperture (28) and an exit aperture which therebetween define the passage, the exit aperture, in use, being in register with the container.

3. A valve according to claim 2 wherein the actuator is substantially cylindrical and is mounted substantially within the body and arranged for unidirectional rotation therein, the actuator including a through bore, which may, by rotation of the actuator, be located in register with both the entry aperture and the exit aperture simultaneously.

4. A valve according to any preceding claim wherein the valve includes a release mechanism (34) which, upon actuation, permits the actuator to be moved from the second position to the transport position, the release mechanism, in use, being operable only upon removal of the valve from the container.

5. A valve according to any preceding claim wherein the valve includes means for certifying the location/time/date or the like at which the sample was taken.

6. A valve according to claim 5 wherein the certifying means comprises a chamber provided in the actuator, the chamber being adapted to carry a token; and a marking device (58) mounted about the body and operable to apply a mark to the token, the chamber being accessible to the marking device only when the actuator is in the first position.

7. A valve according to claim 6 wherein the chamber is located, in use, internally of the body, the body being provided with an access aperture (30) with which the chamber may be brought into register by rotation of the actuator into the first position.

8. A valve according to any preceding claim wherein, when the passage is occluded, an airtight seal is established between the valve and the container.

9. A valve according to any preceding claim further comprising a lock (46) adapted to selectively permit or prevent movement of the actuator.

10. A sampling apparatus comprising a valve according to any preceding claim, and a container (68) engagable therewith.

11. An apparatus according to claim 10 wherein the body is releasably engagable with the container.

12. An apparatus according to claim 10 or 11 comprising a seal (72) which is securable, in use, between the valve and the container, the seal being adapted to prevent or indicate removal of the valve from the container.

13. An apparatus according to any of claims 10 to 12 wherein the coupling and the actuator are interengagable such as to permit the coupling to be locked to the body when the actuator is in the first position.

14. An apparatus according to any of claims 10 to 13 wherein the actuator includes a key (40) which projects outwardly of the body, and the coupling includes a corresponding keyway (54) in which the key is located when the coupling is engaged to the body.

15. An apparatus according to any of claims 10 to 14 wherein the coupling is substantially cylindrical such as, in use, to be engaged substantially about the body in order to prevent access to the body.

16. An apparatus according to claim 13 or 15 wherein the coupling has an open end through which, in use, the body is inserted, and a closed end in which the keyway is located.

17. An apparatus according to any of claims 10 to 16, when dependant on any of claims 7 to 9, wherein the coupling covers the access aperture when the coupling is engaged to the body, the marking device comprising a stamp (60) mounted to the coupling about an opening (66) therein, which opening is in register with the access aperture when the coupling is engaged to the body, such that the stamp may be passed through the access aperture and contact the token, in order to apply the mark to the token.

18. An apparatus according to any of claims 10 to 17 wherein the container is in the form of a hollow truncated cone.

19. An apparatus according to any of claims 10 to 18 wherein the container is substantially transparent or opaque.

## Patentansprüche

1. Probeentnahmeventil (10) zum fluiddichten Transport eines Materials aus einer Einschließung in einen Behälter, wobei das Ventil einen Körper (12) aufweist, der mit dem Behälter und der Einschließung in Eingriff gebracht werden kann, wobei der Körper einen Durchgang, der Zugang zwischen der Einschließung und dem Behälter bereitstellt; ein Betätigungselement (14) in operativer Verknüpfung mit dem Körper enthält, wobei das Betätigungselement für irreversible Bewegung aus einer Transportposition, in der das Betätigungselement den Durchgang zu einer ersten Position verschließt, in der der Durchgang frei liegt, und zur irreversiblen Bewegung aus der ersten Position zu einer zweiten Position eingerichtet ist, in der der Durchgang wieder verschlossen ist;
**dadurch gekennzeichnet, dass** das Ventil Folgendes umfasst:
eine Kupplung (16), die mit dem Körper in Eingriff gebracht werden kann, wenn sich das Betätigungselement in der Transportposition befindet, die an dem Körper verriegelt ist,
wenn sich das Betätigungselement in der ersten Position befindet, und die von dem Körper gelöst werden kann, wenn sich das Betätigungselement in der zweiten Position befindet.

2. Ventil nach Anspruch 1, bei dem der Körper eine Einlassöffnung (28) und eine Auslassöffnung enthält, die zwischen sich den Durchgang begrenzen, wobei die Auslassöffnung in Gebrauch mit dem Behälter ausgerichtet ist.

3. Ventil nach Anspruch 2, bei dem das Betätigungselement im Wesentlichen zylindrisch ist und im Wesentlichen innerhalb des Körpers angebracht und zu Drehung in einer Richtung darin angeordnet ist, wobei das Betätigungselement eine Durchgangsbohrung enthält, die durch Drehung des Betätigungselements gleichzeitig in Ausrichtung sowohl mit der Einlassöffnung als auch der Auslassöffnung angeordnet werden kann.

4. Ventil nach einem vorhergehenden Anspruch, wobei das Ventil einen Freigabemechanismus (34) enthält, der bei Betätigung Bewegen des Betätigungselements aus der zweiten Position in die Transportposition zulässt, wobei der Freigabemechanismus in Gebrauch nur nach Entfernung des Ventils aus dem Behälter betätigt werden kann.

5. Ventil nach einem vorhergehenden Anspruch, wobei das Ventil Mittel zum Zertifizieren des Ortes/ der Zeit/ des Datums oder dergleichen enthält, an dem bzw. zu der die Probe entnommen wurde.

6. Ventil nach Anspruch 5, bei dem das Zertifizierungsmittel eine in dem Betätigungselement vorgesehene Kammer, wobei die Kammer eingerichtet ist, um eine Marke zu tragen; und eine Markiereinrichtung (58) aufweist, die um den Körper herum angebracht ist und betätigt werden kann, um eine Markierung auf der Marke anzubringen, wobei die Kammer für die Markiereinrichtung nur zugänglich ist, wenn sich das Betätigungselement in der ersten Position befindet.

7. Ventil nach Anspruch 6, bei dem sich die Kammer in Gebrauch innerhalb des Körpers befindet, wobei der Körper mit einer Zugangsöffnung (30) versehen ist, mit der die Kammer durch Drehung des Betätigungselements in die erste Position in Ausrichtung gebracht werden kann.

8. Ventil nach einem vorhergehenden Anspruch, bei dem, wenn der Durchgang verschlossen ist, eine luftdichte Dichtung zwischen dem Ventil und dem Behälter gebildet wird.

9. Ventil nach einem vorhergehenden Anspruch, das weiter einen Verschluss (46) aufweist, der eingerichtet ist, um selektiv Bewegung des Betätigungselements zuzulassen oder zu verhindern.

10. Probeentnahmevorrichtung, die ein Ventil nach einem vorhergehenden Anspruch und einen Behälter (68) aufweist, der mit diesem in Eingriff gebracht werden kann.

11. Vorrichtung nach Anspruch 10, bei der der Körper lösbar mit dem Behälter in Eingriff gebracht werden kann.

12. Vorrichtung nach Anspruch 10 oder 11, die eine Dichtung (72) aufweist, welche in Gebrauch zwischen dem Ventil und dem Behälter befestigt werden kann, wobei die Dichtung eingerichtet ist, um Entfernung des Ventils von dem Behälter zu verhindern oder anzuzeigen.

13. Vorrichtung nach einem der Ansprüche 10 bis 12, bei der die Kupplung und das Betätigungselement so miteinander in Eingriff gebracht werden können, um Verriegelung der Kupplung an dem Körper zuzulassen, wenn sich das Betätigungselement in der ersten Position befindet.

14. Vorrichtung nach einem der Ansprüche 10 bis 13, bei der das Betätigungselement einen Keil (40) enthält, der von dem Körper nach außen vorsteht, und die Kupplung eine entsprechende Keilnut (54) enthält, in der sich der Keil befindet, wenn die Kupplung mit dem Körper in Eingriff steht.

15. Vorrichtung nach einem der Ansprüche 10 bis 14, bei der die Kupplung im Wesentlichen zylindrisch ist, um so in Gebrauch im Wesentlichen um den Körper herum in Eingriff zu stehen, um Zugang zu dem Körper zu verhindern.

16. Vorrichtung nach Anspruch 13 oder 15, bei der die Kupplung ein offenes Ende, durch das in Gebrauch der Körper eingeführt wird, und ein geschlossenes Ende aufweist, in dem sich die Keilnut befindet.

17. Vorrichtung nach einem der Ansprüche 10 bis 16 wenn abhängig von einem der Ansprüche 7 bis 9, bei der die Kupplung die Zugangsöffnung bedeckt, wenn die Kupplung mit dem Körper in Eingriff steht, wobei die Markiereinrichtung einen Stempel (60) aufweist, der an der Kupplung um eine Öffnung (66) in derselben herum angebracht ist, welche Öffnung mit der Zugangsöffnung ausgerichtet ist, wenn die Kupplung mit dem Körper in Eingriff steht, so dass der Stempel durch die Zugangsöffnung geführt werden und die Marke berühren kann, um die Markierung auf der Marke anzubringen.

18. Vorrichtung nach einem der Ansprüche 10 bis 17, bei der der Behälter in Form eines hohlen Kegelstumpfes vorliegt.

19. Vorrichtung nach einem der Ansprüche 10 bis 18, bei der der Behälter im Wesentlichen durchsichtig oder undurchsichtig ist.

## Revendications

1. Vanne d'échantillonnage (10) pour le transport étanche aux fluides d'une matière depuis une enceinte jusque dans un contenant, la vanne comprenant un corps (12) pouvant être mis en prise avec le contenant et pouvant être mis en prise avec l'enceinte, le corps comportant un passage qui permet l'accès entre l'enceinte et le contenant ; un actionneur (14) en association opérationnelle avec le corps, l'actionneur étant adapté pour se déplacer de façon irréversible d'une position de transport à laquelle l'actionneur obstrue le passage à une première position à laquelle le passage est exposé, et pour se déplacer de façon irréversible de la première position à une seconde position à laquelle le passage est à nouveau obstrué;
**caractérisée en ce que** la vanne comprend
un raccord (16) pouvant être mis en prise avec le corps quand l'actionneur se trouve sur la position de transport, verrouillé avec le corps quand l'actionneur se trouve sur la première position, et détachable du corps quand l'actionneur se trouve sur la seconde position.

2. Vanne selon la revendication 1, dans laquelle le corps comporte une ouverture d'entrée (28) et une ouverture de sortie qui définissent entre elles le passage, l'ouverture de sortie, durant l'utilisation, étant alignée avec le contenant.

3. Vanne selon la revendication 2, dans laquelle l'actionneur est sensiblement cylindrique et est monté sensiblement à l'intérieur du corps et agencé pour tourner unidirectionnellement dans celui-ci, l'actionneur comportant un alésage traversant, lequel peut, par rotation de l'actionneur, être aligné avec à la fois l'ouverture d'entrée et l'ouverture de sortie simultanément.

4. Vanne selon l'une quelconque des revendications précédentes, dans laquelle la vanne comporte un mécanisme de libération (34) qui, à son actionnement, permet à l'actionneur d'être déplacé de la seconde position à la position de transport, le mécanisme de libération, durant l'utilisation, étant actionnable uniquement lorsque la vanne est retirée du contenant.

5. Vanne selon l'une quelconque des revendications précédentes, dans laquelle la vanne comporte un moyen pour certifier la position/l'heure/la date ou information assimilée à laquelle l'échantillon a été prélevé.

6. Vanne selon la revendication 5, dans laquelle le moyen de certification comprend une chambre aménagée dans l'actionneur, la chambre étant adaptée pour porter un jeton ; et un dispositif de marquage (58) monté autour du corps et exploitable pour appliquer une marque sur le jeton, la chambre étant accessible par le dispositif de marquage uniquement quand l'actionneur se trouve à la première position.

7. Vanne selon la revendication 6, dans laquelle la chambre est située, durant l'utilisation, à l'intérieur du corps, le corps étant doté d'une ouverture d'accès (30) avec laquelle la chambre peut être alignée par rotation de l'actionneur sur la première position.

8. Vanne selon l'une quelconque des revendications précédentes, dans laquelle, quand le passage est obstrué, un joint étanche à l'air est établi entre la vanne et le contenant.

9. Vanne selon l'une quelconque des revendications précédentes, comprenant en outre un verrou (46) adapté pour sélectivement permettre ou empêcher le mouvement de l'actionneur.

10. Appareil d'échantillonnage comprenant une vanne selon l'une quelconque des revendications précédentes, et un contenant (68) pouvant être mis en prise avec celle-ci.

11. Appareil selon la revendication 10, dans lequel le corps peut être mis en prise de façon détachable avec le contenant.

12. Appareil selon la revendication 10 ou 11, comprenant un joint (72) qui peut être fixé, durant l'utilisation, entre la vanne et le contenant, le joint étant adapté pour empêcher ou indiquer le retrait de la vanne du contenant.

13. Appareil selon l'une quelconque des revendications 10 à 12, dans lequel le raccord et l'actionneur peuvent être mutuellement mis en prise de façon à permettre le verrouillage du raccord avec le corps quand l'actionneur se trouve à la première position.

14. Appareil selon l'une quelconque des revendications 10 à 13, dans lequel l'actionneur comporte une clavette (40) qui fait saillie vers l'extérieur du corps, et le raccord comporte un chemin de clavette correspondant (54) dans lequel repose la clavette quand le raccord est mis en prise avec le corps.

15. Appareil selon l'une quelconque des revendications 10 à 14, dans lequel le raccord est sensiblement cylindrique de telle sorte que, durant l'utilisation, il soit mis en prise sensiblement autour du corps pour empêcher l'accès au corps.

16. Appareil selon la revendication 13 ou 15, dans lequel le raccord a une extrémité ouverte à travers laquelle, durant l'utilisation, le corps est inséré, et une extrémité fermée dans laquelle se trouve le chemin de clavette.

17. Appareil selon l'une quelconque des revendications 10 à 16, dépendantes de l'une quelconque des revendications 7 à 9, dans lequel le raccord couvre l'ouverture d'accès quand le raccord est mis en prise avec le corps, le dispositif de marquage comprenant un poinçon (60) monté sur le raccord autour d'une ouverture (66) dans celui-ci, laquelle ouverture est alignée avec l'ouverture d'accès quand le raccord est mis en prise avec le corps, de telle sorte que le poinçon puisse être passé à travers l'ouverture d'accès et fasse contact avec le jeton, afin d'appliquer la marque sur le jeton.

18. Appareil selon l'une quelconque des revendications 10 à 17, dans lequel le contenant a la forme d'un cône tronqué creux.

19. Appareil selon l'une quelconque des revendications 10 à 18, dans lequel le contenant est sensiblement transparent ou opaque.
